# EUROPEAN PATENT APPLICATION

(11) **EP 4 800 393 A1**
(43) Date of publication of application: **02.09.2026**
(21) Application number: 25160732.1
(22) Date of filing: 27.02.2025
(51) Int. Cl.: G01N 33/574

(54) **METHOD FOR BREAST CANCER BIOMARKER DETECTION, A MICROFLUIDIC SYSTEM, A KIT AND USES THEREOF**

(30) Priority: 26.02.2025 PT 2025120077
(71) Applicant: Universidade de Aveiro, 3810-193 Aveiro (PT); Inesc Microsistemas E Nanotecnologias - Instituto de Engenharia de Sistemas e Computadores para os Microsistemas e as Nanotecnologias, 1000-029 Lisboa (PT)
(72) Inventor: OSÓRIO DE ALMEIDA COELHO E SILVA, Ana Francisca, 3810-193 AVEIRO (PT); MARQUES MENDES, Maria Silvina, 3810-193 AVEIRO (PT); FREIRE MARTINS, Mara Guadalupe, 3810-193 AVEIRO (PT); CHU, Virginia, 1000-029 LISBOA (PT)
(74) Representative: Patentree

(57) **Abstract**

The present disclosure relates to point-of-care diagnostics, specifically to a microfluidic sandwich immunoassay for the detection of breast cancer biomarkers (for ex: HER2 biomarker) in biological samples, utilizing dual ionic liquid all-aqueous extraction for enhanced sensitivity and fluorescence-based quantification. This solution enables highly sensitive, low-volume, and point-of-care diagnostics, offering an unexpectedly lower limit of detection (LoD) compared to conventional methods.

## Description

### TECHNICAL FIELD

The present disclosure relates to the field of bioanalytical chemistry and medical diagnostics, specifically to the development of an integrated method that combines dual ionic liquid all-aqueous extraction with a microfluidic immunoassay for point-of-care analysis of cancer biomarkers. More particularly, the invention is designed for the detection of protein biomarkers of breast cancer in biological samples, preferably human serum, offering superior sensitivity and specificity by minimizing matrix effects.

The present invention relates to point-of-care diagnostics, specifically to a microfluidic sandwich immunoassay for the detection of HER2 biomarkers in biological samples, utilizing dual ionic liquid all-aqueous extraction for enhanced sensitivity and fluorescence-based quantification.

### BACKGROUND

Breast cancer is one of the leading causes of cancer-related mortality worldwide. The early and accurate detection of biomarkers, such as human epidermal growth factor receptor-type 2 (HER2), is critical for timely diagnosis and treatment decisions.

Cancer remains one of the leading global health challenges, with an estimated 20 million new cases and 9.7 million cancer-related deaths in 2022, according to the latest global cancer statistics. Projections indicate that by 2050, the global incidence of cancer will rise to 35 million new cases, with low development index countries expected to experience a relative increase of 142%. Among all cancer types, breast cancer stands out as the second most common and fatal, highlighting the urgent need for better disease management and early detection strategies (Bray et al. 2024 "Global cancer statistics 2022: GLOBOCAN estimates of incidence and mortality worldwide for 36 cancers in 185 countries" CA Cancer J Clin. 74:229-263).

Clinical biomarkers, such as estrogen receptor (ER), progesterone receptor (PR), and human epidermal growth factor receptor 2 (HER2), play an increasingly critical role in breast cancer management, aiding in early diagnosis, prognosis, and therapy selection (Gamble et al. 2021 "Determining breast cancer biomarker status and associated morphological features using deep learning" Commun Med 1:14). Approximately 10-30% of human breast cancers are characterized by the amplification and/or overexpression of the HER2 gene located on chromosome 17q12. As a result, accurate assessment of HER2 status is essential for determining appropriate treatment strategies, including targeted therapies (Iqbal and lqbal 2014 "Human Epidermal Growth Factor Receptor 2 (HER2) in Cancers: Overexpression and Therapeutic Implications" Mol Biol Int 2014:1-9). Currently, the evaluation of HER2 status is carried out through two main techniques: immunohistochemistry (IHC) for protein overexpression and fluorescent in situ hybridization (FISH) for gene amplification (Ahn et al. 2020; "HER2 status in breast cancer: changes in guidelines and complicating factors for interpretation" J Pathol Transl Med 54(1):34-44). Although these methods are highly reliable and widely used, they are time-consuming, labour-intensive, and require trained personnel to conduct complex procedures (Lottner et al 2005 "Simultaneous Detection of HER2/Neu Gene Amplification and Protein Overexpression in Paraffin-Embedded Breast Cancer" J Pathol 205(5):577-584; Gohring et al. 2010 "Detection of HER2 Breast Cancer Biomarker Using the Opto-Fluidic Ring Resonator" Biosensor. Sens Actuators B Chem 146(1):226-230). These limitations hinder their widespread use, especially for decentralized diagnostics and resource-constrained settings, such as countries with low to medium human development indices or remote rural areas in nations with higher human development indices, where access to specialized equipment and skilled personnel is limited.

Recent evidence on HER2 testing highlights the clinical usefulness and practicality of measuring HER2 levels directly in human serum samples, particularly for patients with HER2-positive breast cancer. These patients typically exhibit circulating HER2 levels higher than 15 ng/mL, which require highly sensitive techniques capable of accurately quantifying such low concentrations (Monteiro et al. 2017 "Platform Based on Image Acquisition for HER2 Antigen Detection" Nanotechnology 28(4):045206; Lam et al. 2012 "Challenges in the clinical utility of the serum test for HER2 ECD" Biochim Biophys Acta Rev Cancer 1826(1):199-208). Moreover, serum-based testing offers a less invasive alternative to tissue biopsies, enabling repeated biomarker monitoring over time. This is especially relevant in resource-limited settings where access to tissue samples may be difficult (Moelans et al. 2011 "Current Technologies for HER2 Testing in Breast Cancer" Crit Rev Oncol Hemat 80(3):380-392; Leyland-Jones et al. 2011 "Serum HER2 Testing in Patients with HER2-Positive Breast Cancer: The Death Knell Tolls" Lancet Oncol 12(3):286-295).

The most employed technique for circulating HER2 quantification is enzyme-linked immunosorbent assay (ELISA). While ELISA is effective, it involves complex, multistage protocols that require highly qualified personnel, along with costly specialized reagents and equipment (Loo et al. 2011 "Highly Sensitive Detection of HER2 Extracellular Domain in the Serum of Breast Cancer Patients by Piezoelectric Microcantilevers" Anal Chem, 83(9):3392-3397; Wu and Qu 2015 "Cancer Biomarker Detection: Recent Achievements and Challenges" Chem Soc Rev 44(10):2963-2997). To address these analytical challenges, microfluidic lab-on-a-chip devices are particularly well-suited for point-of-care analysis. Their advantages include miniaturization, automation, rapid response times, and the ability to use reduced sample and reagent volumes, making them more accessible than traditional laboratory methods (Tavakoli et al. 2022 "Microfluidic Platforms Integrated with Nano-Sensors for Point-of-Care Bioanalysis" TrAC Trend Anal Chem 157:116806).

Biological samples have a complex composition that often interferes with the reliable analysis of low-abundance proteins, including biomarkers. In human serum, the most abundant proteins are human serum albumin (HSA) and immunoglobulin G (IgG), which together represent approximately 80% of the protein content. These can cause significant interference in bioanalysis, especially when detecting low-abundance biomarkers, presenting a challenge for clinical applications (da Costa et al. 2017 "How low can you go? A current perspective on low-abundance proteomics" TrAC Trends Anal Chem 93:171-182). To mitigate these interferences, several techniques are employed for the removal of high-abundance proteins such as IgG and HSA. The most common methods include protein precipitation using salts, polymers, and organic solvents, as well as affinity depletion with columns that use specific ligands to bind and remove HSA and IgG. However, these techniques have inherent limitations that can lead to false positive or false negative results. Such limitations include incomplete removal of high-abundance proteins, lack of specificity in targeting these proteins, potential impacts on the integrity and stability of biomarkers, frequent losses of biomarkers, multi-step procedures, and the reliance on volatile organic solvents or costly resources (Polaskova et al. 2010 "High-abundance Protein Depletion: Comparison of Methods for Human Plasma Biomarker Discovery" Electrophoresis 31(3):471-482). Therefore, there is still a need for the development of a method that can effectively reduce matrix effects while enabling efficient analysis of cancer biomarkers. This method should also address environmental and economic concerns, providing a more sustainable and accessible solution for bioanalysis (López-Lorente et al. 2022 "The ten principles of green sample preparation" TrAC Trend Anal Chem 148:116530).

The present invention intends to develop an integrated method that combines biological sample preparation with microfluidic devices for the efficient analysis of breast cancer biomarkers, with potential for point-of-care applications, especially in resource-constrained settings. The method resorts to dual ionic liquid all-aqueous extraction for sample preparation, seamlessly integrated with microfluidic detection, offering several technological benefits: (i) high selectivity, enabled by the use of two ionic liquids specifically tailored to remove high-abundance serum proteins and extract target biomarkers in separate phases; (ii) mild, all-aqueous extraction conditions that preserve protein biomarkers, avoiding false negative results; and (iii) economic and environmental advantages through a simplified analytical workflow, free from multi-step processes, volatile organic solvents, and expensive reagents.

All-aqueous extraction strategies, formulated with two aqueous phases of polymers, salts, or surfactants, have been used for the mild extraction of protein biomarkers from biological samples (Raymond et al. 1994 "Separation of alkaline phosphatase isoforms with and without intact glycan-phosphatidylinositol anchors in aqueous polymer phase systems" Clin Chim Acta 227:111-120; Garza-Madrid et al. 2010 "Potential of Aqueous Two-Phase Systems constructed on flexible devices: Human serum albumin as proof of concept" Process Biochem 45(7):1082-1087). Specifically, these methods have been used to remove albumin from human biological samples, such as serum, prior to bioanalysis using laboratory-based techniques, though with modest efficiency (Silva and Arruda 2009 "An aqueous two-phase system as a strategy for serum albumin depletion" Talanta 77(3):985-990). These strategies have also shown potential to simultaneously remove HSA and IgG prior to analysing lung cancer protein biomarkers in serum samples using techniques such as ELISA (Rosa et al. 2024 "Enhancing Biomarker Detection in Human Serum for Lung Cancer Diagnosis: Aqueous Biphasic Systems for Simultaneous Depletion of High-Abundance Proteins and Efficient Extraction of CYFRA 21-1" Adv Sample Prep 10:100116; Mendes et al. 2023 "Improved accuracy in pentraxin-3 quantification assisted by aqueous biphasic systems as serum pretreatment strategies" Int J Biol Macromol 253:127540). Despite these advancements, all-aqueous extraction strategies remain limited for clinical applications and point-of-care detection, where high selectivity and efficiency are crucial.

Ionic liquids, particularly when combined with strong salting-out agents, have proven to be versatile and tunable tools for designing all-aqueous extraction strategies, enhancing both efficiency and selectivity (Rosa et al. 2023 "Tailored pretreatment of serum samples and biomarker extraction afforded by ionic liquids as constituents of aqueous biphasic systems" Sep Purif Technol 322: 124248). These systems have also been combined with microfluidic devices for the detection of prostate-specific antigen (PSA) (Flora et al. 2023 "Combined Use of Ionic Liquid-Based Aqueous Biphasic Systems and Microfluidic Devices for the Detection of Prostate-Specific Antigen" Biosensors 13(3):334). Using these systems, it was possible to remove over 99% of HSA and IgG. However, this complete removal of high-abundance proteins also resulted in a significant loss of PSA (about 28%), which is undesirable in clinical applications as it can lead to false negative results (Rosa et al. 2023 "Tailored pretreatment of serum samples and biomarker extraction afforded by ionic liquids as constituents of aqueous biphasic systems" Sep Purif Technol 322: 124248). In contrast to the use of a single ionic liquid combined with a strong salting-out agent, the present invention introduces the innovative approach of using two ionic liquids in all-aqueous extraction. This approach allows for a more customizable polarity range in the sample preparation process, with the goal of enhancing extraction efficiency and selectivity. The aim is to provide a more efficient and accurate method for preparing human serum samples prior to the analysis of the breast cancer biomarker HER2 using a microfluidic immunoassay.

Document WO2020089797A1 describes a biological sample pretreatment technique based on an ionic liquid and a strong salting-out agent, specifically developed for the differential diagnosis of prostate cancer.

Document BR102017026617A2 discloses a concentration kit for adrenal cancer diagnosis, using an ionic liquid and inorganic salts. Both documents focus on human urine, rather than human serum, as in the present invention.

Document PT116172B proposes a pretreatment method for human serum using a single ionic liquid and a salt buffer, while focusing on the extraction of lactate dehydrogenase as a general cancer biomarker. Unlike previous reports, the present invention introduces a novel sample preparation strategy based on dual ionic liquid all-aqueous extraction, specifically designed for breast cancer detection and monitoring. Furthermore, whereas the mentioned documents focus on purifying and/or concentrating target biomarkers for analysis using laboratory-reliant chromatographic techniques, the present invention integrates this sample preparation with portable microfluidic immunoassays, making point-of-care applications feasible.

These facts are disclosed in order to illustrate the technical problem addressed by the present disclosure.

### GENERAL DESCRIPTION

The present disclosure relates to a novel analytical method for point-of-care detection of breast cancer biomarkers in biological samples. The analytical workflow integrates a sample preparation procedure based on dual ionic liquid all-aqueous extraction with biomarker analysis using a microfluidic immunoassay. Dual ionic liquid all-aqueous extraction is employed to minimize matrix effects before bioanalysis, improving both efficiency and selectivity.

The present disclosure improves the detection limits of microfluidic immunoassays compared to direct serum analysis, enabling highly sensitive and specific bioanalysis at the point of care.

The present solution relates to an integrated method for the point-of-care detection of breast cancer biomarkers, particularly HER2, using dual ionic liquid all-aqueous extraction and a microfluidic immunoassay. The dual ionic liquid extraction efficiently removes high-abundance proteins while selectively isolating the biomarker, significantly reducing matrix effects. The extracted HER2 is then analysed via a fluorescent microfluidic sandwich immunoassay, achieving higher sensitivity and specificity compared to conventional methods. The method is designed for rapid, portable applications, making it particularly suitable for point-of-care diagnostics in resource-constrained settings.

An integrated method for point-of-care detection of breast cancer biomarkers in a biological sample, comprising dual ionic liquid all-aqueous extraction for surprisingly selective biomarker isolation, wherein this extraction method unexpectedly removes high-abundance proteins, thereby enhancing detection sensitivity; a microfluidic sandwich immunoassay is employed for biomarker detection, utilizing capture and detection antibodies for surprisingly precise breast cancer biomarker quantification; with a fluorescence-based quantification of the biomarker enables unexpectedly high sensitivity and specificity.

The method of the present disclosure achieves a surprisingly lower limit of detection (LoD) compared to direct serum analysis and is specifically adapted for unexpectedly efficient point-of-care applications in resource-limited environments.

The objective of the present invention is to provide a sensitive and specific method for the point-of-care analysis of breast cancer biomarkers, particularly human epidermal growth factor receptor-type 2 (HER2), in biological samples, such as human serum. The developed analytical workflow integrates a sample preparation step based on dual ionic liquid all-aqueous extraction with a microfluidic immunoassay. This method uses dual ionic liquid all-aqueous extraction to efficiently remove high-abundance proteins from serum, while enabling the selective extraction of HER2. The extracted biomarker is then analysed using a microfluidic sandwich immunoassay coupled to fluorescence detection.

The integrated method of the present disclosure offers a significantly lower limit of detection (LoD) when compared to direct analysis of serum samples. Additionally, by minimizing matrix effects, the method improves the sensitivity and specificity of biomarker analysis, making it applicable in resource-constrained environments for rapid and reliable point-of-care tests.

The present invention applies to the field of bioanalysis, specifically the extraction and detection of cancer biomarkers from complex biological samples. More particularly, it discloses an integrated method that combines dual ionic liquid all-aqueous extraction with a microfluidic immunoassay to enable the efficient analysis of breast cancer biomarkers, such as HER2, in human serum. This approach ensures selective removal of high-abundance proteins and maximizes HER2 recovery, significantly improving analytical sensitivity and specificity. While developed for breast cancer diagnostics, the method is adaptable to other biomarkers and biological fluids, enabling broader biomedical applications.

Existing methods for biomarker extraction and detection rely on conventional sample preparation techniques, such as solid-phase extraction and affinity depletion, which often suffer from limitations such as incomplete removal of interfering proteins, loss of target biomarkers, and labour-intensive workflows. In contrast, this invention introduces a dual ionic liquid system that operates under mild, all-aqueous conditions, preventing protein losses while enabling high selectivity for both the removal of high-abundance proteins (e.g., HSA and IgG) and the efficient extraction of HER2 in a single step. This improves downstream detection and prevents false negatives, a major limitation in current point-of-care tests.

The method consists of two integrated steps. First, a dual ionic liquid all-aqueous extraction, which comprises two ionic liquids that work synergistically to selectively precipitate high-abundance proteins at the interphase while simultaneously extracting HER2 into a distinct phase. Second, a microfluidic immunoassay for detection following extraction. The biomarker-enriched phase is analysed using a microbead-based sandwich immunoassay within a microfluidic chip, allowing for sensitive and specific detection through fluorescence-based readout, even at low concentrations.

This approach combines a range of advantages that are unprecedented in any existing sample preparation method. These include high selectivity and biomarker recovery, superior analytical sensitivity, mild and sustainable conditions, and compatibility with point-of-care applications.

The disclosed method is poised to revolutionize biomarker detection beyond HER2 and breast cancer applications.

By integrating sample preparation with microfluidic detection, the present disclosure provides an accessible tool for biomarker analysis, enabling early disease detection, monitoring, and better patient management.

The present disclosure described the combination of dual ionic liquid all-aqueous extraction and a microfluidic immunoassay to unexpectedly enhance detection sensitivity of breast cancer biomarker, like HER2.

A method for the detection of a target breast cancer HER2 biomarker in a biological sample, comprising:
mixing the biological sample with an ionic liquid mixture (preferably dual ionic liquid aqueous solution) for liquid-liquid extraction (this is an all-aqueous extraction) for isolating the target breast cancer biomarker;
centrifuge the mixture and collect the supernatant (or the liquid biomarker-enriched phase);
introduce the obtained liquid biomarker-enriched phase in a microfluidic immunoassay comprising a breast cancer capture antibody and a breast cancer-specific antibody conjugated with a fluorescent marker for enabling the selective capture and detection of the target breast cancer biomarker,
wherein the ionic liquid mixture comprises triisobutylmethylphosphonium tosylate ([P_{*i*(444)1}][TsO]) and cholinium dihydrogen phosphate ([Ch][H₂PO₄]).

The dual ionic liquid all-aqueous extraction method of the present disclosure efficiently removes 100% of IgG and 84% of HSA at the solid interphase. Unlike previous methods that resulted in significant biomarker losses, this approach ensures high breast cancer biomarker, namely HER2, recovery yield (97%), preventing false negatives in clinical analyses. Additionally, the method eliminates the need for volatile organic solvents, ensuring compatibility with HER2 stability, while simplifying sample pretreatment into a single step for the removal of high-abundance proteins and biomarker extraction.

In an embodiment for better results, comprising a solvent-to-sample weight ratio from 8:1 to 10:1; preferably 9:1.

In an embodiment for better results, wherein the extraction time ranges from 5-15 minutes, preferably 10 minutes.

In an embodiment for better results, the ionic liquid mixture comprises 35-40% (w/w) of [Pᵢ₍₄₄₄₎₁][TsO] and 25-35% (w/w) of [Ch][H₂PO_{4]} based on the total extraction sample (based on the total all-aqueous extraction system composition, this is ionic liquid mixture (may further include a buffer, etc..),and a biological sample making up the remaining percentage to reach 100% (w/w).

In an embodiment for better results, the ionic liquid mixture comprises 36-38 (w/w) of [P_{*i*(444)1}][TsO] and 28-32% (w/w) of [Ch][H₂PO_{4]} based on the total all-aqueous extraction system composition; more preferably 35% (w/w) of [Pᵢ₍₄₄₄₎₁][TsO] and 30% (w/w) of [Ch][H₂PO₄].

In an embodiment for better results, the ionic liquid mixture comprises 35% (w/w) [P_{*i*(444)1}][TsO], 30% (w/w) [Ch][H₂PO₄], 25% (w/w) saline buffer, and a biological sample making up the remaining percentage to reach 100% (w/w).

In an embodiment for better results, the ionic liquid mixture comprises a saline buffer and two ionic liquids that can be phosphonium-based or ammonium-based, this is triisobutylmethylphosphonium tosylate ([P_{*i*(444)1}][TsO]) and cholinium dihydrogen phosphate ([Ch][H₂PO₄]).

In an embodiment for better results, the extracted solution is centrifuged at 3500-5000 rpm for 10-20 minutes.

In an embodiment for better results, the biological sample is human serum, blood, plasma, urine, saliva, tears, sweat, vaginal secretions, breast milk.

In an embodiment for better results, the concentration of the target breast cancer biomarker in the sample is superior to 14.06 ng/mL.

Another aspect of the present disclosure relates to a microfluidic immunoassay, in particular a microfluidic sandwich immunoassay, for the detection of a breast cancer biomarker, in particular HER2, comprising:
a microfluidic platform with defined reaction chambers and fluidic channels for controlled reagent flow;
a suitable capture antibody immobilized on a solid-phase surface within the microfluidic channel;
wherein the solid-phase surface comprises: a primary antibody to capture the target biomarker and a secondary antibody conjugated to a fluorescent marker for binding to the captured target biomarker for the detection of target biomarker, forming a sandwich complex upon biomarker binding; in particular with the detection antibody being present at a 2:1 of a weight ratio relative to the capture antibody; a fluorescence-based detection system for quantification.

In an embodiment for better results, the solid-phase surface comprises protein G beads.

In an embodiment for better results, the detection antibody is an anti-HER2 antibody; namely an anti-HER2 antibody selected from the following list: trastuzumab, pertuzumab, polyclonal anti-HER2 antibody, anti-HER2 monoclonal antibody clone SP3, or mixtures thereof; preferably wherein detection antibody is a polyclonal anti-HER2 antibody.

In an embodiment for better results, the fluorescent marker is selected from a list consisting of: Alexa Fluor Dyes, Cy^{™} Dyes, fluorescein isothiocyanate, tetramethylrhodamine isothiocyanate, or mixtures thereof.

In an embodiment for better results, microfluidic device may include additional detection technologies, for electrochemical or colorimetric detection, to enhance portability, sensitivity, and versatility.

Another aspect of the present disclosure relates to a kit for the detection of a breast cancer biomarker in a biological sample comprising microfluidic immunoassay according of the present disclosure and a dual ionic liquid comprising triisobutylmethylphosphonium tosylate ([P_{*i*(444)1}][TsO]) and cholinium dihydrogen phosphate ([Ch][H₂PO₄]).

Use of a ionic liquid mixture for detecting breast cancer biomarker of a biological sample, as an extractor or extraction agent of said biomarkers, wherein the dual ionic liquid comprises triisobutylmethylphosphonium tosylate ([P_{*i*(444)1}][TsO]) and cholinium dihydrogen phosphate ([Ch][H₂PO₄]).

### BRIEF DESCRIPTION OF THE DRAWINGS

The following figures provide preferred embodiments for illustrating the disclosure and should not be seen as limiting the scope of invention.
Figure 1: Schematic representation of the analytical workflow, integrating the sample preparation step based on dual ionic liquid all-aqueous extraction with microfluidic detection.
Figure 2: Recovery efficiency of high-abundance serum proteins after sample preparation with dual ionic liquid all-aqueous extraction.
Figure 3: Recovery yield of HER2 from serum after sample preparation with dual ionic liquid all-aqueous extraction.
Figure 4: Fluorescence intensity of HER2 detection using a microbead-based sandwich immunoassay in a microfluidic device.
Figure 5: Sensitivity curves for HER2 analysis across various sample matrices.

### DETAILED DESCRIPTION

The present disclosure relates to point-of-care diagnostics, specifically to a microfluidic sandwich immunoassay for the detection of breast cancer biomarkers (for ex: HER2 biomarkers) in biological samples, utilizing dual ionic liquid all-aqueous extraction for enhanced sensitivity and fluorescence-based quantification. This solution enables highly sensitive, low-volume, and point-of-care diagnostics, offering an unexpectedly lower limit of detection compared to conventional methods.

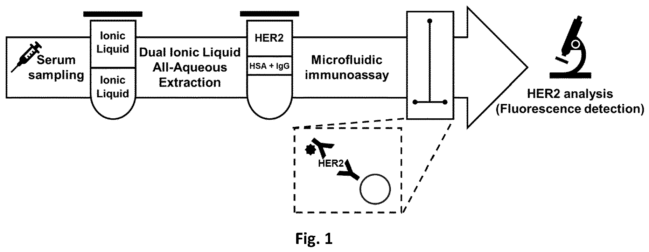

The present disclosure relates to an integrated method for the efficient analysis of the breast cancer biomarker HER2 using a dual ionic liquid all-aqueous extraction system coupled with a microfluidic immunoassay (Figure 1). This approach surprisingly enables the selective removal of high-abundance proteins from human serum and the efficient extraction of HER2, facilitating its subsequent detection in a microfluidic device suitable for point-of-care applications.

In an embodiment, the method begins with sampling and subsequent preparation of the biological sample using dual ionic liquid all-aqueous extraction. The ionic liquids are selected based on their polarity and ability to form two phases in an aqueous medium. Their proportions are optimized to enhance biomarker extraction and remove high-abundance proteins. The selected ionic liquids are triisobutylmethylphosphonium tosylate ([P_{*i*(444)1}][TsO]) and cholinium dihydrogen phosphate ([Ch][H₂PO₄]). This combination enables selective protein removal through precipitation at the interphase, maximizing HER2 extraction into the top phase and minimizing losses during preparation.

In an embodiment, the extraction mixture composition for a total of 1 g is: 35% (w/w) [P_{*i*(444)1}][TsO], 30% (w/w) [Ch][H₂PO₄], 10% (w/w) commercial human serum (Sigma-Aldrich H4522 - Lot #SLCD4040), and 25% (w/w) phosphate buffer saline (PBS, acquired from Sigma-Aldrich). The extraction solvent is composed of 0.35 g of [P_{*i*(444)1}][TsO], 0.30 g of [Ch][H₂PO_{4]} and 0.25 g of phosphate buffer saline. The components are weighed together with 0.1 g of biological sample, agitated to ensure complete dissolution, and centrifuged at 3500 rpm for 10 minutes. After centrifugation, the top and bottom phases are separated from the solid interphase, which is enriched in high-abundance proteins, and collected for analysis.

In an embodiment, to assess the efficiency of protein removal, IgG and HSA quantification is performed in each phase using size-exclusion high-performance liquid chromatography (SE-HPLC). The Chromaster HPLC system (VWR Hitachi) is used, equipped with a binary pump, column oven, and auto-sampler maintained at 25 °C and 10 °C, respectively. The analytical column is Protein KW-802.5 (Shodex, 8 mm × 300 mm), coupled to a DAD detector set to 280 nm. The mobile phase is a 50 mM phosphate buffer (NaH₂PO₄/Na₂HPO₄) and 0.3 M NaCl solution, with an isocratic flow rate of 0.5 mL/min. The removal efficiency is calculated as the ratio of protein mass precipitated at the interphase to the initial protein mass in human serum.

In an embodiment, the HER2 extraction from human serum is evaluated by spiking serum with HER2 at 15 ng/mL, corresponding to the clinical cutoff for HER2 in serum (Asgeirsson et al. 2007 "Serum epidermal growth factor receptor and HER2 expression in primary and metastatic breast cancer patients" Breast Cancer Res 9(6):R75). HER2 quantification is performed using a commercial ELISA kit (Human HER2-total ELISA Kit RAB0173 Lot # 1220J0287 from Sigma Aldrich), following the manufacturer's provided protocol. The recovery yield is determined by comparing the mass of HER2 extracted into the top phase to the initial spiked mass.

Another aspect of the present disclosure relates to a microfluidic immunoassay for HER2 detection. After sample preparation, the extracted biomarker is analysed using a microfluidic platform with a sandwich-type immunoassay coupled with fluorescence detection. The device, designed for small-volume handling, is suitable for point-of-care applications. In the assay, antibodies specific to HER2 are immobilized on a solid phase. The target HER2 in the serum is captured by these antibodies, and a secondary antibody conjugated to a fluorescent marker binds to the captured HER2, enabling sensitive detection of even low HER2 concentrations, critical for cancer detection. Fluorescence detection is used, with the fluorescence signal correlating to HER2 concentration in the sample.

In an embodiment, the microfluidic structures in polydimethylsiloxane (PDMS) are fabricated using soft lithography, involving the creation of a rigid aluminium mask, the production of an SU-8 mold, and the manufacturing of the PDMS structures. The device consists of two sections of different heights (a shallower channel with a height of 20 µm and a taller chamber with a height of 100 µm), optimizing the packing of Protein G beads, which enables the sandwich immunoassay for HER2 detection. Fluid handling is precisely controlled by a syringe pump, and image acquisition is performed using a fluorescence microscope.

In an embodiment, the microfluidic sandwich immunoassay begins by packing Protein G microbeads into the microchannel at a flow rate of 8 µL/min for 3 minutes, followed by a 3-minute wash with PBS at the same flow rate. A 100 µg/mL solution of capture anti-HER2 antibody is then introduced at a flow rate of 0.5 µL/min for 10 minutes to immobilize the antibodies on the beads. After immobilization, a 1 mg/mL IgG solution is added at the same flow rate for 10 minutes to block non-specific binding. To detect HER2, solutions spiked with HER2 at concentrations ranging from 5 to 25 ng/mL are introduced at 0.5 µL/min for 10 minutes. Negative controls containing 0 ng/mL HER2 are included for baseline measurements. Following the HER2 samples, a 100 µg/mL solution of Alexa 430-labeled detection anti-HER2 antibody is pumped at 0.5 µL/min for 10 minutes. Between each step, PBS is flowed at 5 µL/min for 1 minute to remove unbound molecules and reduce background noise.

Sensitivity curves are generated by plotting fluorescence intensity against HER2 concentration for each condition: HER2 in PBS, untreated human serum, and HER2 in the top phase of the dual ionic liquid all-aqueous extraction system. The LoD for each condition is determined as the concentration at which the fluorescence signal exceeds three standard deviations above the baseline signal from the negative control.

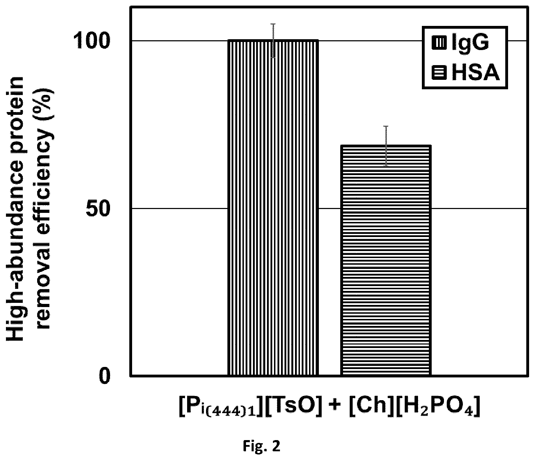

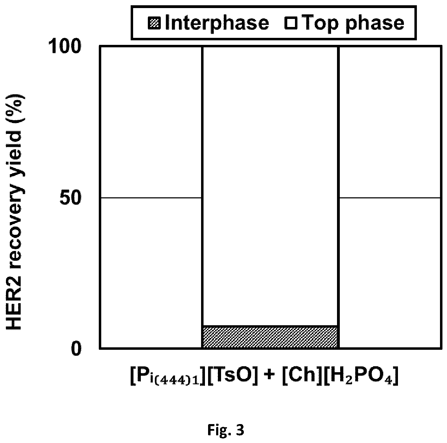

The present disclosure describes a novel sample preparation and detection strategy that delivers a combination of advantages not found in any single method. The dual ionic liquid all-aqueous extraction system efficiently removes 100% of IgG and 84% of HSA at the solid interphase (Figure 2). Unlike previous methods that resulted in significant biomarker losses, this approach ensures high HER2 recovery yield (97%), preventing false negatives in clinical testing (Figure 3). Additionally, the method eliminates the need for volatile organic solvents, ensuring compatibility with HER2 stability, while simplifying sample pretreatment into a single step for the removal of high-abundance proteins and biomarker extraction.

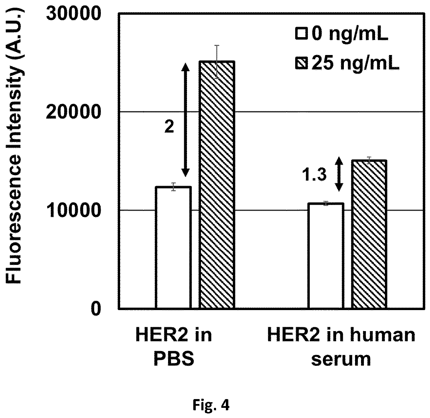

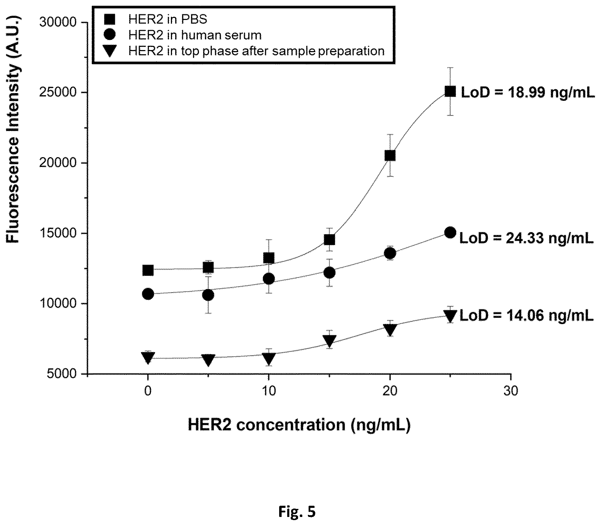

The present disclosure further demonstrates the successful development of a microfluidic device for the sensitive and specific detection of HER2 in human serum using a sandwich immunoassay with Protein G beads as a solid support. The assay was first evaluated in PBS and then in human serum samples spiked with HER2. A signal-to-noise ratio of 2 was observed between positive and negative controls in PBS, which decreased to 1.3 in serum due to matrix effects (Figure 4). To minimize these effects, the dual ionic liquid all-aqueous extraction step was integrated into the analytical workflow, lowering the LoD for HER2 from 24.33 ng/mL in untreated serum and 18.99 ng/mL in PBS to 14.06 ng/mL, significantly improving sensitivity (Figure 5). The microfluidic immunoassay enables on-site HER2 detection, making it suitable for resource-limited settings and decentralized healthcare.

Overall, this integrated method offers high selectivity, efficiency, and sensitivity in detecting HER2, with reduced matrix effects and environmental benefits. The system is suitable for point-of-care applications, enabling early detection of HER2-positive breast cancer, monitoring disease progression, and assessing treatment responses. While demonstrated for HER2, the method can be adapted to other cancer biomarkers or non-cancer biomarkers in various body fluids. It has the potential to enhance early cancer diagnosis, reduce reliance on complex and resource-intensive laboratory techniques, and improve healthcare delivery in resource-limited settings.

The term "comprising" whenever used in this document is intended to indicate the presence of stated features, integers, steps, components, but not to preclude the presence or addition of one or more other features, integers, steps, components or groups thereof.

As used in the specification and claims, the singular forms "a", "an" and "the" include plural references unless the context clearly dictates otherwise. For example, the term "a sample" includes a plurality of samples, including mixtures thereof.

Whenever the term "at least," "greater than," or "greater than or equal to" precedes the first numerical value in a series of two or more numerical values, the term "at least," "greater than" or "greater than or equal to" applies to each of the numerical values in that series of numerical values. For example, greater than or equal to 1, 2, or 3 is equivalent to greater than or equal to 1, greater than or equal to 2, or greater than or equal to 3.

The terms "determining," "measuring," "evaluating," "assessing," "assaying," and "analysing" are often used interchangeably herein to refer to forms of measurement. The terms include determining if an element is present or not (for example, detection). These terms can include quantitative, qualitative or quantitative and qualitative determinations. Assessing can be relative or absolute. "Detecting the presence of" can include determining the amount of something present in addition to determining whether it is present or absent depending on the context.

Where ranges are given, endpoints are included. Furthermore, it is to be understood that unless otherwise indicated or otherwise evident from the context and/or the understanding of one of ordinary skill in the art, values that are expressed as ranges can assume any specific value within the stated ranges in different embodiments of the invention, to the tenth of the unit of the lower limit of the range, unless the context clearly dictates otherwise. It is also to be understood that unless otherwise indicated or otherwise evident from the context and/or the understanding of one of ordinary skill in the art, values expressed as ranges can assume any subrange within the given range, wherein the endpoints of the subrange are expressed to the same degree of accuracy as the tenth of the unit of the lower limit of the range.

The disclosure should not be seen in any way restricted to the embodiments described and a person with ordinary skill in the art will foresee many possibilities to modifications thereof.

The above-described embodiments are combinable.

The following claims further set out particular embodiments of the disclosure.

## Claims

1. A method for the detection of a target breast cancer HER2 biomarker in a biological sample, comprising:
mixing the biological sample with an ionic liquid mixture (preferably dual ionic liquid aqueous solution) for liquid-liquid extraction (or all-aqueous extraction) for isolating the target breast cancer biomarker;
centrifuge the mixture and collect the supernatant (or liquid biomarker-enriched phase);
introduce the obtained liquid biomarker-enriched phase in a microfluidic immunoassay comprising a breast cancer capture antibody and a breast cancer-specific antibody conjugated with a fluorescent marker for enabling the selective capture and detection of the target breast cancer biomarker,
wherein the ionic liquid mixture comprises triisobutylmethylphosphonium tosylate ([P_{*i*(444)1}][TsO]) and cholinium dihydrogen phosphate ([Ch][H₂PO₄]).

2. The method according to the previous claim, comprising a solvent-to-sample weight ratio of from 8:1 to 10:1; preferably 9:1.

3. The method according to any of the previous claims, wherein the extraction time ranges from 5-15 minutes, preferably 10 minutes.

4. The method according to any of the previous claims, wherein the ionic liquid mixture comprises 35-40% (w/w) of [P_{*i*(444)1}][TsO], 25-35% (w/w) of [Ch][H₂PO_{4]} and a biological sample making up the remaining percentage to reach 100% (w/w)

5. The method according to any of the previous claims, wherein the ionic liquid mixture comprises 36-38% (w/w) of [P_{*i*(444)1}][TsO] and 28-32% (w/w) of [Ch][H₂PO₄]; more preferably 35% (w/w) of [Pᵢ₍₄₄₄₎₁][TsO] and 30% (w/w) of [Ch][H₂PO₄].

6. The method according to any of the previous claims, wherein the ionic liquid mixture further comprises a saline buffer.

7. The method according to any of the previous claims, wherein the ionic liquid mixture comprises 35% (w/w) [P_{*i*(444)1}][TsO], 30% (w/w) [Ch][H₂PO_{4]} and 25% (w/w) saline buffer, and a biological sample making up the remaining percentage to reach 100% (w/w).

8. The method according to any of the previous claims, wherein the obtained mixture is centrifuged at 3500-5000 rpm for 10-20 minutes.

9. The method according to any of the previous claims, wherein the biological sample is human serum, blood, plasma, urine, saliva, tears, sweat, vaginal secretions, breast milk.

10. The method according to any of the previous claims, wherein the concentration of the target breast cancer in the sample is superior to 14.06 ng/mL.

11. A microfluidic immunoassay for detecting a breast cancer biomarker, in particular HER2, comprising:
a microfluidic platform with defined reaction chambers and fluidic channels for controlled reagent flow;
a suitable capture antibody immobilized on a solid-phase surface within the microfluidic channel;
wherein the solid-phase surface comprises: a primary antibody to capture the target biomarker and a secondary antibody conjugated to a fluorescent marker for binding to the captured target biomarker for the detection of target biomarker, forming a sandwich complex upon biomarker binding; in particular with the detection antibody being present at a 2:1 of a ratio relative to the capture antibody;
a fluorescence-based detection system for quantification;
in particular wherein the solid-phase surface comprises protein G beads.

12. The microfluidic sandwich immunoassay according to the previous claims 10-11 detection antibody is a HER2 antibody; namely a HER2 antibody selected from the following list: trastuzumab, pertuzumab, polyclonal anti-HER2 antibody, anti-HER2 monoclonal antibody clone SP3, or mixtures thereof; preferably wherein detection antibody is a polyclonal anti-HER2 antibody and/or wherein the fluorescent marker is selected from a list consisting of: Alexa Fluor Dyes, Cy^{™} Dyes, fluorescein isothiocyanate, tetramethylrhodamine isothiocyanate, or mixtures thereof.

13. A kit for the detection of a breast cancer biomarker in a biological sample comprising microfluidic sandwich immunoassay according to any of the claims 10-12 and a ionic liquid mixture comprising triisobutylmethylphosphonium tosylate ([P_{*i*(444)1}][TsO]) and cholinium dihydrogen phosphate ([Ch][H₂PO₄]).

14. Use of a ionic liquid mixture for detecting breast cancer biomarker of a biological sample, as an extractor or extraction agent of said biomarkers, wherein the ionic liquid mixture comprises triisobutylmethylphosphonium tosylate ([P_{*i*(444)1}][TsO]) and cholinium dihydrogen phosphate ([Ch][H₂PO₄]).

15. A composition for a liquid-liquid extraction of breast cancer biomarker comprising an ionic liquid mixture, wherein the ionic liquid mixture comprises 35-40% (w/w) of [Pᵢ₍₄₄₄₎₁][TsO] and 25-35% (w/w) of [Ch][H₂PO_{4]} based on the total all-aqueous extraction system composition.
